(19) Europäisches Patentamt
European Patent Office
Office européen des brevets

(11) EP 2 692 742 A1

(12) # EUROPEAN PATENT APPLICATION

published in accordance with Art. 153(4) EPC

(43) Date of publication:
**05.02.2014 Bulletin 2014/06**

(21) Application number: **12764051.4**

(22) Date of filing: **29.03.2012**

(51) Int Cl.:
***C08F 2/44*** (2006.01) ***A61K 9/14*** (2006.01)
***A61K 31/198*** (2006.01) ***A61K 31/401*** (2006.01)
***A61K 31/405*** (2006.01) ***A61K 31/4172*** (2006.01)
***A61K 47/32*** (2006.01) ***A61P 31/04*** (2006.01)
***A61P 35/00*** (2006.01) ***A61P 35/02*** (2006.01)
***C08F 120/42*** (2006.01) ***C08F 251/00*** (2006.01)
***C08F 283/04*** (2006.01)

(86) International application number:
**PCT/JP2012/058354**

(87) International publication number:
**WO 2012/133648 (04.10.2012 Gazette 2012/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2011 JP 2011078781**

(71) Applicant: **NanoCAME Co., Ltd.**
**Yokohama-shi, Kanagawa 244-0003 (JP)**

(72) Inventor: **SHIROTAKE, Shoichi**
**Kanagawa 244-0003 (JP)**

(74) Representative: **Wills, Andrew Jonathan**
**Mewburn Ellis LLP**
**33 Gutter Lane**
**London**
**EC2V 8AS (GB)**

(54) # NOVEL PREPARATION TECHNIQUE FOR HIGHER-ORDER STRUCTURE THAT EXHIBITS ANTI-CELLULAR EFFECT

(57) Disclosed is a novel means by which cyanoacrylate polymer particles still more useful as an antibacterial agent, an anticancer agent, etc., than conventional particles. The method for production of cyanoacrylate polymer particles according to the present invention comprises anionically polymerizing a cyanoacrylate monomer(s) in the presence of at least one selected from the group consisting of amino acids, amino acid derivatives, and oligomers and polymers thereof, and substantially in the absence of a saccharide and substantially in the absence of a polysorbate. Cyanoacrylate nanoparticles containing an amino-based molecule(s) exhibit their cell-damaging activity via specific adhesion to cells, and effectively inhibit the growth of cancer cells and bacteria. Their cell-damaging activity can be further increased by producing the nanoparticles by the above-mentioned novel production method.

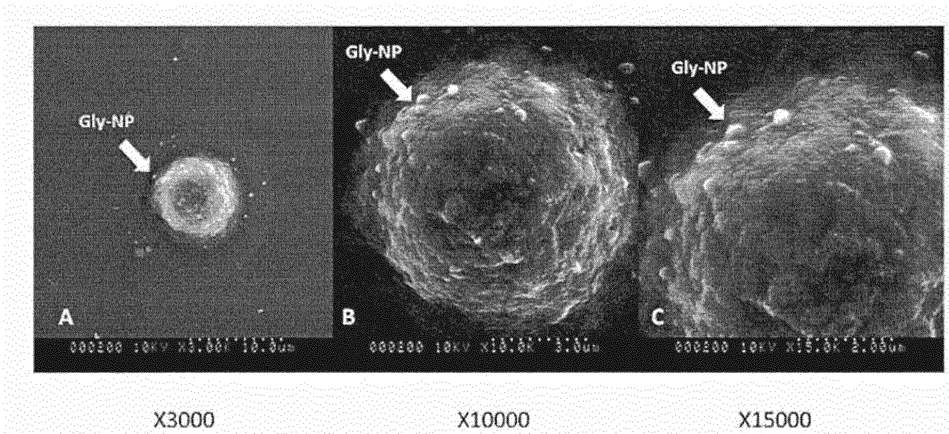


Fig.7

EP 2 692 742 A1

## Description

### TECHNICAL FIELD

The present invention relates to a novel method for production of cyanoacrylate nanoparticles, a cell-damaging agent comprising as an effective ingredient nanoparticles which contain at least one selected from the group consisting of amino acids, amino acid derivatives, and oligomers and polymers thereof, and a technique for modulating its cell-damaging activity.

### BACKGROUND ART

In order to improve the effect of pharmaceuticals by drug delivery system (DDS) or by sustained release, studies of nano-encapsulation of drugs are now under way. For example, DDS in which a drug is encapsulated in cyanoacrylate polymer particles is known (Patent Documents 1, 2 and Non-patent Document 1). The present inventors also have disclosed a method for producing cyanoacrylate polymer particles with little irregularity in particle diameter, antibiotic-containing particles, and plasmid-containing particles (Patent Documents 3 to 5). In a conventional method for synthesizing polymer particles, a saccharide(s) and/or a polysorbate(s) is(are) made to be present in the polymerization reaction system. These past studies aimed at DDS or sustained release of drugs.

Thereafter, the present inventor found that cyanoacrylate polymer particles *per se* have an antibacterial activity against Gram-positive bacteria (Patent Document 6). Nano-sized polymer particles specifically adhere to the cell wall of Gram-positive bacteria to cause bacteriolysis. They exhibit their antibacterial activity by a mechanism completely different from the mechanism of antibiotics, and are effective even against multidrug-resistant bacteria such as MRSA and VRE.

Furthermore, the present inventor found that amino acid-containing cyanoacrylate polymer particles have an anticancer activity (Patent Document 7). Amino acid *per se* does not have special pharmacologic actions, but the anticancer activity of polymer particles is improved by incorporating an amino acid(s) into the particles. The anticancer activity is altered depending on which kind of amino acid the particles contain, and thus the particles are applicable to various kinds of cancers by selecting the kind of amino acid to be contained therein.

### PRIOR ART DOCUMENTS

### PATENT DOCUMENTS

Patent Document 1: JP H 11-503148 A
Patent Document 2: JP 2002-504526 A
Patent Document 3: JP 2008-127538 A
Patent Document 4: WO 2008/126846
Patent Document 5: JP 2008-208070 A
Patent Document 6: WO 2009/084494
Patent Document 7: WO 2010/101178

### NON-PATENT DOCUMENTS

Non-patent Document 1: Christine Vauthier et al., Adv. Drug Deliv. Rev., 55, 519-548 (2003)

### SUMMARY OF THE INVENTION

### PROBLEMS TO BE SOLVED BY THE INVENTION

An object of the present invention is to provide a novel means by which cyanoacrylate polymer particles still more useful as an antibacterial agent, anticancer agent and the like than conventional ones can be provided.

## MEANS FOR SOLVING THE PROBLEMS

The present inventor has intensively studied to find that initiation and stabilization of anionic polymerization can be achieved by using an amino acid(s) alone, without using a saccharide and without using a polysorbate, and that any of neutral, acidic and basic amino acids, and any of linear, aromatic, imino, and sulfur-containing structures have an effect of initiation/stabilization of polymerization, thereby completing a novel method for synthesizing cyanoacrylate nanoparticles. The present inventor has also studied on amino acid-containing particles, whose use as an anticancer drug was known, and found that they can exhibit an antibacterial activity higher than the nanoparticles containing no amino acids, that they can exhibit their antibacterial activity against not only Gram-positive bacteria but also Gram-negative bacteria, and that their antibacterial activity can be further increased by producing amino acid-containing particles by the above-mentioned novel synthesis method. Moreover, the present inventor has found that the activity of cyanoacrylate polymer particles can be understood as a manifestation of the action on cells the particles have (cell-damaging activity) as an antibacterial activity and an anticancer activity, and that the level of cell-damaging activity of the particles can be modulated by selecting the kind of amino acid to be contained in the particles and/or by selecting a method for synthesis of the nanoparticles, thereby completing the present invention.

That is, the present invention provides a method for production of cyanoacrylate polymer particles, said method comprising anionically polymerizing a cyanoacrylate monomer(s) in the presence of at least one selected from the group consisting of amino acids, amino acid derivatives, and oligomers and polymers thereof, and substantially in the absence of a saccharide and substantially in the absence of a polysorbate. The present invention also provides a cell-damaging agent comprising as an effective ingredient cyanoacrylate polymer particles whose average particle diameter is less than 1000 nm, said particles containing at least one selected from the group consisting of amino acids, amino acid derivatives, and oligomers and polymers thereof. The present invention further provides a method for modulating the cell-damaging activity of the above-described cell-damaging agent of the present invention, characterized in that the cell-damaging activity of the cyanoacrylate polymer particles is modulated by at least one of: selection of the kind of an amino acid(s), an amino acid derivative(s), and/or an oligomer(s) and/or a monomer(s) thereof; and selection of the polymerization process of cyanoacrylate polymer particles. The present invention still further provides an antibacterial agent comprising as an effective ingredient cyanoacrylate polymer particles having an average particle diameter of less than 1000 nm, wherein said particles contain at least one selected from the group consisting of amino acids, amino acid derivatives, and oligomers and polymers thereof, and substantially do not contain an active antibacterial ingredient. The present invention still further provides an agent for therapy and/or prophylaxis of a cancer(s), comprising as an effective ingredient particles produced by the above-described method of the present invention.

## EFFECTS OF THE INVENTION

By the present invention, a novel method for producing nano-sized cyanoacrylate polymer particles with little irregularity in particle diameter was provided. In addition, a novel means by which the level of the activity of cyanoacrylate nanoparticles against cells can be modulated was provided. Particles which contain at least one selected from the group consisting of amino acids, amino acid derivatives, and oligomers and polymers thereof have a higher antibacterial activity than the known antibacterial nanoparticles (Patent Document 6), and can exhibit still higher antibacterial effect against both resistant and sensitive bacteria. The particles can also exhibit their antibacterial activity against not only Gram-positive bacteria but also Gram-negative bacteria. Their antibacterial activity can be further increased by carrying out the polymerization reaction of cyanoacrylate just using at least one selected from amino acids, amino acid derivatives and oligomers and polymers thereof, without using a saccharide and without using a polysorbate.

It has been known that amino acid-containing particles have a high anticancer activity and that the particles are applicable to various kinds of cancers by selecting the kind of amino acid to be contained therein (Patent Document 7). The level of their anticancer activity can be controlled by carrying out the polymerization of cyanoacrylate just using at least one selected from amino acids, amino acid derivatives, and oligomers and polymers thereof. Therefore, not only the types of the cancer to be treated or prevented, but also application of personalized medicine to many individual patients, can be increased. There have been no drugs before whose antibacterial and anticancer activities can be understood as the action on cells. The present invention is a significant invention, which can increase the potential of cyanoacrylate nanoparticles.

## BRIEF DESCRIPTION OF THE DRAWINGS

Fig. 1 is a graph showing the antibacterial activity (minimum inhibitory concentration (MIC)) of the particles produced by Dex-Amino acid incorporating system in Production Example 1 against various staphylococcal strains.

Fig. 2 is a graph showing the antibacterial activity (minimum inhibitory concentration (MIC)) of the particles produced by Dex-Amino acid incorporating system in Production Example 1 against various enterococcal strains.

Fig. 3 is a graph showing the antibacterial activity (minimum inhibitory concentration (MIC)) of the amino acid-containing nanoparticles produced in Production Example 1 and Production Example 2 against various staphylococcal strains.

Fig. 4 is a graph showing the antibacterial activity (minimum inhibitory concentration (MIC)) of the amino acid-containing nanoparticles produced in Production Example 1 and Production Example 2 against various enterococcal strains.

Fig. 5 shows SEM images of VRE NCTC12201 treated with the amino acid-containing nanoparticles (0.01 HCl-Gly+Dex70) produced in Production Example I (1-, 3- and 6-hour after the particle treatment).

Fig. 6 is a graph showing the survival rate of various cancer cell lines treated with the amino acid-containing nanoparticles produced in Production Example 1 and Production Example 2.

Fig. 7 shows SEM images of an H9 cell treated with the amino acid-containing nanoparticles (0.01 HCl-Gly+Dex70) produced in Production Example 1 (2-hour after the particle treatment).

MODE FOR CARRYING OUT THE INVENTION

In the novel synthesis method of nanoparticles of the present invention, at least one selected from the group consisting of amino acids, amino acid derivatives, and oligomers and polymers thereof (which are also collectively hereinafter referred to as "amino-based molecule") is used for initiation and stabilization of polymerization of cyanoacrylate instead of conventionally used saccharides and polysorbates. Cyanoacrylate nanoparticles which contain an amino-based molecule(s) can be produced by anionically polymerizing a cyanoacrylate monomer(s) in the presence of at least one selected from amino acids, amino acid derivatives, and oligomers and polymers thereof, and substantially in the absence of a saccharide and substantially in the absence of a polysorbate, preferably in the complete absence of a saccharide and in the complete absence of a polysorbate.

In the present invention, the term "amino acid" refers to a compound which has an amino group(s) and a carboxy group(s) in the molecule, and as generally defined, the term also includes imino acids, which are cyclic secondary amine compounds in which a hydrogen of an amino group is substituted with another moiety in the molecule. Representative examples of the amino acid which may be used in the present invention include the 20 kinds of $\alpha$-amino acids which constitute natural proteins, but the examples are not restricted thereto and also include $\beta$-, $\gamma$- and $\delta$-amino-based molecules. Specific examples thereof include, but not limited to, arginine, histidine, lysine, aspartic acid, glutamic acid, alanine, glycine, leucine, valine, isoleucine, serine, threonine, phenylalanine, tryptophan, tyrosine, cystine or cysteine, glutamine, asparagine, proline, methionine, $\beta$-alanine, $\gamma$-aminobutyric acid (GABA; neurotransmitter), carnitine, $\gamma$-aminolevulinic acid, $\gamma$-aminovaleric acid and the like.

The term amino acid "derivative" refers to a compound having a structure in which any of the groups in the amino acid as defined above is(are) modified or substituted. Amino acid derivatives which naturally occur as biogenic substances may be usually preferably used in the present invention. Specific examples of the amino acid derivative which may be used include, but not limited to, creatine (an arginine derivative, 1-methylguanidinoacetic acid), ornithine (an arginine derivative, product of urea cycle), thyroxine (triiodothyronine; T4, belonging to aromatic amino acids), desmosine (a component of corneum elastin and collagen; having a structure in which side chains of three allysine molecules and a side chain of one lysine molecule are bound), hydroxyproline and hydroxylysine (a component of gelatin and collagen), phosphoserine (an ester of serine and phosphoric acid; a component of casein), theanine (a tee component, glutamic acid derivative), kainic acid (an anthelmintic component of Corsican weed), tricholomic acid (a component of Lyophyllum), sarcosine (a component of yolks, ham, legumes; N-methylglycine) and the like.

In the present invention, the term amino acid "oligomer" refers to an oligopeptide in which not more than 10 amino acid residues are bound by a peptide bond(s), and the term amino acid "polymer" refers to a polypeptide in which not less than 11 amino acid residues are bound by peptide bonds. Both may comprise not only an amino acid(s) but also an amino acid derivative(s) as residues. The upper limit of the number of the residues of polypeptide is not restricted, and for example, may be not more than 500 residues. A polypeptide of 11-100 residues, 11-50 residues, 11-30 residues, 1-20 residues, or 11-15 residues may be preferably used.

An oligopeptide may be used more preferably than a polypeptide. In particular, a oligopeptide of 2-7 residues, 2-5 residues, or 2 or 3 residues may be more preferably used.

In the following Examples, it is demonstrated that nano-sized (less than 1000 nm) cyanoacrylate polymer particles can be synthesized by using any of the 20 kinds of $\alpha$-amino acids (i.e. arginine, histidine, lysine, aspartic acid, glutamic acid, alanine, glycine, leucine, valine, isoleucine, serine, threonine, phenylalanine, tryptophan, tyrosine, cystine or cysteine, glutamine, asparagine, proline, methionine), without using a saccharide and without using a polysorbate. It is demonstrated that nanoparticles can be produced by using any of neutral, acidic and basic amino acids, or any of linear, aromatic, imino, sulfur-containing structures, without using a saccharide and without using a polysorbate. Thus,

not only the 20 kinds of α-amino acids actually used in the Examples below, but also the above-described other amino acids and amino acid derivatives may be used in the synthesis of nanoparticles. In addition, oligopeptides and polypeptides may also be used in the synthesis of nanoparticles, since they also have amino acid structures in the molecule.

As the cyanoacrylate monomer(s), an alkyl cyanoacrylate monomer(s) (the alkyl group is preferably a $C_1$-$C_8$ alkyl) is(are) preferred, and especially preferred is *n*-butyl-2-cyanoacrylate (nBCA) represented by the following formula, which is conventionally used as an adhesive for wound closure in the field of surgery.

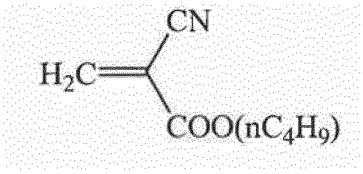

According to the novel synthesis method of nanoparticles, neither a saccharide nor a polysorbate is used in the polymerization reaction, and the polymerization initiating/stabilizing effects exhibited by the amino acid structure are utilized. Thus, in cases where particles are produced by this method, amino-based molecule-containing particles substantially do not contain, preferably do not contain at all, a saccharide, and substantially do not contain, preferably do not contain at all, a polysorbate. For example, nano-sized cyanoacrylate polymer particles with a uniform particle diameter can be obtained by dissolving an amino-based molecule(s) which should be incorporated into the particles in a solvent; adding thereto a cyanoacrylate monomer(s) under stirring; and continuing stirring the mixture to allow the polymerization reaction to proceed. The particles may contain one kind of amino-based molecule, or two or more kinds of amino-based molecules may be dissolved in a solvent to incorporate the molecules into the particles at the same time. As described later, the kind of the amino-based molecule contained in the particles influences on the cell-damaging activity the particles have, and the level of cell-damaging activity can be modulated by selection of the kind of the amino-based molecule.

As for possible modes of incorporation of amino-based molecule(s) into particles, it is thought that the -COO group in the amino acid structure is covalently bound to a carbon at the ethylene end of cyanoacrylate, or that an amino-based molecule(s) is(are) attached to the particles noncovalently. In the present invention, the mode of incorporation of amino-based molecule(s) into particles is not restricted as long as the amino-based molecule(s) is(are) contained in cyanoacrylate polymer particles.

When a cyanoacrylate monomer(s) is(are) anionically polymerized in the presence of an amino-based molecule (s), the amino-based molecule(s) is(are) incorporated in the polymer particles not only by adhesion to the particles but also by a covalent bond as described above. By utilizing a functional group on the amino-based molecule(s) bound to the polymeric moiety via covalent bond, amino-based molecule-containing particles can be immobilized on a desired material via covalent bond, which is advantageous for immobilization of the particles onto a medical material. The ratio of incorporation of the amino-based molecule(s) into the particles obtained by the above-described method is usually about 20% to about 65%. However, the incorporation ratio may be out of this range as long as the particles have a cell-damaging activity. The ratio of incorporation of amino-based molecule(s) may be calculated by measuring the absorbance of a liquid which has passed through a filter obtained in the filter washing steps after the polymerization at an appropriate wavelength, determining the amount of amino-based molecule(s) in the filter-passing liquid based on the measured absorbance, and by calculating the ratio according to the following equation:

Amount of Incorporated Amino-Based Molecule(s) = (Amount of amino-based molecule(s) added) - (Amount of amino-based molecule(s) in filter-passing liquid)

Ratio of Amino-Based Molecule Incorporation (%) = Amount of incorporated amino-based molecule(s) / Amount of amino-based molecule(s) added x 100

As a solvent for the polymerization, an aqueous solvent which is based on water (e.g. water, aqueous solution of lower alcohol and the like) may be used, and water is usually preferably used in the case of production of amino-based molecule-containing particles. Because the anionic polymerization is initiated by hydroxide ion, the velocity of polymerization is influenced by pH of the reaction solution. When pH of the reaction solution is high, polymerization proceeds rapidly because of a high concentration of hydroxide ion. When pH is low, polymerization proceeds slowly. In the case of production of amino-based molecule-containing particles, an appropriate polymerization velocity is usually attained under an acidic condition of about pH 1.5 to 3.0. The acid added to the reaction solution in order to acidify it is

not restricted, and hydrochloric acid may be preferably used as it does not have a bad influence on the reaction and vaporizes after the reaction. The concentration of hydrochloric acid is not restricted, and may be appropriately selected within the range of about 0.0005 N to 0.5 N.

The concentration of the monomer(s) in the polymerization reaction solution at the start of the reaction is not restricted, and is usually about 0.5 v/v% to 2.0 v/v%, preferably about 0.8 v/v% to 1.2 v/v%.

The concentration of the amino-based molecule(s) in the polymerization reaction solution at the start of the reaction is not restricted, and is usually about 0.1 w/v% to 3 w/v%. In the case where amino-based molecule-containing particles are produced by a conventional method, not by the novel synthesis method of nanoparticles, the concentration may be lower than the above-described concentration.

The reaction temperature is not restricted, and the reaction is preferably carried out at room temperature because it is simple. As for the reaction time, the reaction velocity varies depending on pH of the reaction solution, the kind of the solvent and the like, and therefore the reaction time is appropriately selected depending on these factors. The reaction time is usually, but not limited to, about 10 minutes to 5 hours, preferably about 30 minutes to 4 hours. As the obtained amino acid-containing particles are usually used as neutral particles, it is preferred to neutralize the reaction solution by adding thereto a base such as aqueous sodium hydroxide solution or the like after completion of the reaction.

Processes of producing cyanoacrylate polymer particles containing a desired substance by the conventional method using a saccharide(s) and/or a polysorbate(s) are known as described in Patent Document 3, Patent Document 4 (antibacterial drug-containing particles), Patent Document 5 (plasmid-containing particles), Patent Document 7 (amino acid-containing particles). In cases where particles containing a desired amino-based molecule(s) are produced in accordance with these conventional methods, the following steps may be carried out. That is, for example, an amino-based molecule(s) to be contained in particles and at least one selected from saccharides and polysorbates are dissolved in a solvent; a cyanoacrylate monomer(s) is(are) added thereto while stirring the mixture; and the polymerization reaction is allowed to proceed while stirring the mixture as appropriate.

Saccharides are not restricted, and may be any of monosaccharides having a hydroxyl group(s) (e.g. glucose, mannose, ribose, fructose and the like), disaccharides having a hydroxyl group(s) (e.g. maltose, trehalose, lactose, sucrose and the like) and polysaccharides having a hydroxyl group(s) (e.g. dextran, mannan and the like). These saccharides may be either a cyclic form or a chain form, and in case of cyclic form, saccharides may be either a pyranose form or a furanose form. Saccharides have various isomers, and any of such isomers may be used in the present invention. Polysorbates are not restricted, and may be any of the known Tween series surfactants such as polyoxyethylene sorbitan monolaurate (trade name: Tween 20), polyoxyethylene sorbitan monooleate (trade name: Tween 80) and the like. Any one of monosaccharides, disaccharides and polysaccharides and polysorbates may be used alone, or two or more of them may be used in combination. Among the saccharides and polysorbates described above, glucose, dextran and Tween 20 (trade name) are preferred, and dextran is especially preferred. As for the polymerization degree of dextran, dextran having an average molecular weight of about 70,000 or more is preferred. The upper limit of the molecular weight of dextran is not restricted, and the molecular weight is usually about 500,000 or less.

In the conventional method, the concentration of saccharide(s) and/or polysorbate(s) (in the case where a plurality of kinds thereof are used, the total concentration thereof) is not restricted, and is usually about 0.5% to 10%, preferably about 0.75% to 7.5%. As used herein, the concentration of saccharides means w/v%, and the concentration of polysorbates means v/v%. For example, in the case where one saccharide is used alone, the above-described concentration ranges mean "0.5 w/v% to 10 w/v%" and "0.75 w/v% to 7.5 w/v%", respectively. In the case where 5 w/v% of saccharide and 1 v/v% of polysorbate are used in combination, the total concentration thereof is expressed as 6%. It should be noted that, in the case where a monosaccharide(s) (e.g. glucose) is(are) used alone, the monosaccharide (s) is(are) preferably used at a concentration of about 2.5 w/v% to 10 w/v%.

By the above-described novel synthesis method of nanoparticles or conventional method, nano-sized amino-based molecule-containing particles with an average particle diameter of less than 1000 nm can be easily produced. The lower limit of the particle size is not restricted, and the particle diameter of the particles produced by the above-described polymerization reaction is usually about 7 nm or more. The average particle diameter of the particles is preferably 20 nm to 600 nm, more preferably 50 nm to 550 nm. The size of the particles can be regulated by regulating the concentration of cyanoacrylate monomer(s) in the reaction solution, pH, and/or the reaction time. In the case where at least one selected from saccharides and polysorbates is used as a polymerization initiator/stabilizer, the particle size can also be regulated by changing the concentration and/or the kind of the polymerization initiator/stabilizer (see, Patent Documents 3, 4, etc.). In general, the particle size becomes large in cases where pH of the reaction solution is high, where the reaction is carried out for a longer time, and where the concentration of saccharide(s) in the reaction solution is lowered; whereas, the particle size becomes small in cases where a polysorbate(s) is(are) used as a polymerization initiator/stabilizer. Particles with a desired size can be produced by appropriately combining these reaction conditions.

The electric charge (Zeta potential) of the amino-based molecule-containing particles is not restricted, and usually about -50 mV to 0 mV. "Zeta potential" represents an electric charge of the surface of particles, and is an indicator of the dispersion stability of particles. The particle size and the Zeta potential may be easily measured with, for example,

a commercially available device utilizing a He-Ne laser (such as Zetasizer manufactured by Malvern Inst.UK).

The cell-damaging agent of the present invention comprises as an effective ingredient nano-sized cyanoacrylate polymer particles containing at least one amino-based molecule, which particles can be produced by the above-described methods. The amino-based molecule-containing particles used in the present invention are substantially composed of an amino-based molecule(s) and cyanoacrylate polymer(s) (and, a saccharide(s) and/or a polysorbate(s) in the case where particles are produced by the conventional method), and do not contain a substance known as a cytotoxin. The particles exhibit their cell-damaging activity via specific adhesion to the subject cells. Against bacteria, the particles adhere to the cell wall to induce bacteriolysis (antibacterial activity); and against tumor cells, the particles produce an apoptosis-like response to induce cell death, thereby inhibiting their growth (anticancer activity). The term "cell-damaging activity" as used in the present invention is a concept that includes both an antibacterial activity and an anticancer activity. Therefore, the cell-damaging agent of the present invention may be used as an antibacterial agent and an agent for therapy and/or prophylaxis of a cancer(s). It has been confirmed that the cell-damaging activity the nanoparticles have is not exhibited against normal mammalian cells, and that the nanoparticles have no *in vivo* toxicity.

The level of cell-damaging activity of the particles can be modulated by selection of polymerization processes (whether at least one selected from saccharides and polysorbates is used or not). The particles produced by the novel synthesis method of nanoparticles have a higher cell-damaging activity than amino acid-containing particles produced by the conventional method which contain a saccharide(s) and/or polysorbate(s). Therefore, if it is desired to increase the cell-damaging activity, the anionic polymerization may be carried out substantially in the absence of a saccharide and substantially in the absence of a polysorbate.

The level of the cell-damaging activity of the particles may also be modulated by selection of the kind of amino-based molecule(s) to be contained. Examples of the amino acid by which a higher antibacterial activity can be obtained than the known antibacterial nanoparticles (Patent Document 6) include alanine, glycine, valine, isoleucine, serine, threonine, phenylalanine, tyrosine, tryptophan, aspartic acid, glutamic acid, asparagine, glutamine, histidine and proline. Among these, a still higher antibacterial activity can be obtained by glycine, serine, threonine, glutamic acid, asparagine, histidine and proline, and in particular, an especially high level of antibacterial activity can be obtained by glycine, asparagine and histidine. As for the anticancer activity, the kind of amino acid which produces a high effect varies depending on the kind of cancer as described in Patent Document 7, and therefore various cancers can be dealt with by appropriately selecting the kind of amino acid to be contained in the particles. For example, against malignant lymphomas, glycine-, asparagine-, glutamine- and histidine-containing particles may be especially effective. Against colon cancers, glycine-, asparagine- and histidine-containing particles may be especially effective; against pancreatic cancer cells, glycine- and histidine-containing particles may be especially effective; and against liver cancers, histidine-containing particles may be especially effective. Examples of the amino acid which can especially increase both antibacterial and anticancer activities of the nanoparticles include glycine, asparagine and histidine.

The antibacterial activity of nanoparticles containing no amino acids is disclosed in Patent Document 6. The present inventor has newly found that the antibacterial activity of nanoparticles can be increased by incorporating an amino-based molecule(s) into the particles, that such particles can exhibit their antibacterial activity against not only Gram-positive bacteria but also Gram-negative bacteria, and that their antibacterial activity can be further increased by producing the particles by the novel synthesis method of nanoparticles. The amino-based molecule-containing particles exhibit their antibacterial action in the same manner as the particles of Patent Document 6, i.e., the particles adhere to the bacterial surface (cell wall) to cause bacteriolysis (Fig. 5). The kind of bacteria to be targeted is not restricted, and may be Gram-positive bacteria or Gram-negative bacteria. The amino-based molecule-containing particles are effective against various bacteria including pathogen of animals including human, and especially highly effective against Gram-positive bacteria. Specific examples of Gram-positive bacteria to be targeted include, but not limited to, *Staphylococcus aureus, enterococci, streptococci* (such as *Streptococcus pneumoniae,* oral streptococci, *Streptococcus pyogenes,* bacteria belonging to *Peptostreptococcus*), *Corynebacterium diphtheriae, Propionibacterium acnes,* acid-fast bacilli (such as tubercle bacilli, non-tuberculous mycobacteria) and the like. Specific example of Gram-negative bacteria to be targeted include, but not limited to, *Escherichia coli, Pseudomonas aeruginosa,* salmonellae, *Klebsiella pneumoniae* and the like. Regardless of drug resistance of bacteria, the growth of both sensitive bacteria and multidrug-resistant bacteria can be inhibited. For example, not only in the case of sensitive bacteria such as MSSA (methicillin-sensitive *Staphylococcus aureus*), VSE (vancomycin-sensitive enterococci), sensitive tuberculosis and the like, but also in the case of multidrug-resistant bacteria such as MRSA (methicillin-resistant *Staphylococcus aureus*), VRE (vancomycin-resistant enterococci), multidrug-resistant tuberculosis and the like, bacteriolysis is induced by adhesion of the particles, and thus the bacterial growth is inhibited. Since the antibacterial activity of the amino-based molecule-containing particles is completely different from that of conventional antibiotics, the particles has no risk of occurrence of new multidrug-resistant bacteria as well as can inhibit the growth of the existing multidrug-resistant bacteria, which is very advantageous in the clinical use.

The detailed principle by which specific adhesion of the particles causes bacteriolysis is uncertain. Although the scope of the present invention is not bound by theory, the following is presented relating to the principle. That is, in

the fundamental cell wall synthesis, UDP-MurNAc-pentapeptide is synthesized, and then bound to fatty acids in the cell membrane and thereafter bound to GluNAc to form lipid-MurNAc(GluNAc)-pentapeptide. MurNAc therein is bound to GluNAc existing in a peptidoglycan which is being synthesized, and thus cell wall having a branching structure is built. Cell wall synthesis is executed on the external surface of the cell wall. Against Gram-positive bacteria, by adhesion of the particles to the bacterial surface, cell wall synthesis at the site where the particles have adhered is disturbed and the cell wall is weakened to cause breakage of the cell wall, and hence bacteriolysis occurs, which is presumed from the observations of scanning and transmission electron microscopic images and inhibition of incorporation of radioisotope labeled glucose into the cell wall. Against Gram-negative bacteria, which have an outer membrane at the outer side of the peptidoglycan cell wall, the following is presented relating to the principle. That is, by incorporating an amino acid (s) into nanoparticles, lipophilic amino groups are introduced into the particles, and thus affinity of the particles for the lipid layer of the surface of Gram-negative bacteria increases, which enables the particles to adhere to Gram-negative bacteria.

The nanoparticles used in the present invention substantially do not contain, preferably do not contain at all, an active antibacterial ingredient effective against bacteria. The term "active antibacterial ingredient" means a chemical component which can biochemically affect metabolic pathway and/or physiological functions of bacteria to inhibit their growth, and specifically means a chemical component which can be used for inhibition of bacterial growth such as antibiotics or the like. The term "substantially do not contain" means that particles do not contain any active antibacterial ingredients at all, or that even if they contain an active antibacterial ingredient(s), the amount of the contained active antibacterial ingredient(s) is so small that the growth of bacteria sensitive to the contained antibacterial ingredient(s) cannot be inhibited. In regard to the above-mentioned small amount by which the growth of sensitive bacteria cannot be inhibited, an amount of the active antibacterial ingredient(s) contained in a unit volume of the particles is defined as concentration in particles. When the growth of a sensitive bacterium cannot be inhibited by treating the bacterium with the active antibacterial ingredient(s) in form of not being incorporated into particles at the same concentration as the above-defined concentration in particles, the concentration is considered to be an amount by which the growth of the sensitive bacterium cannot be inhibited.

The anticancer activity of amino acid-containing particles is disclosed in Patent Document 7. The amino acid-containing particles can exhibit their cell-damaging activity against various cancer cells including cervical cancer, T-cell lymphoma, B-cell lymphoma, monocytic leukemia, renal cancer and pancreatic cancer, and also colon cancer and liver cancer as described in the following Examples. It is thought that the same effect can be obtained when particles containing an amino acid derivative(s), oligopeptide(s) or polypeptide(s) are used. On the other hand, when amino acid-containing particles are administered to healthy mice, the mice show no abnormalities and cytotoxicity to normal cells is not detected. Thus, when administering the particles to a living body, they may specifically exhibit a damaging activity against cancer cells present in the living body, and therefore amino-based molecule-containing particles are useful as an agent for therapy and/or prophylaxis of a cancer(s).

In the case where amino-based molecule-containing particles are used in therapy and/or prophylaxis of a cancer (s), the cancer(s) to be treated is(are) not restricted. The particles are applicable to various cancers including uterine cancers (such as cervical cancer), lymphomas (such as non-Hodgkin lymphomas including T-cell lymphoma, B-cell lymphoma, etc.), leukemias (such as monocytic leukemia), renal cancers, pancreatic cancers, colon cancers, liver cancers and the like.

The kind of amino-based molecule which can confer the highest anticancer activity on nanoparticles varies depending on the kind of cancer, and also may vary from patient to patient even if they have the same kind of cancer. In the present invention, various cancers and patients can be dealt with individually optimally by appropriately selecting the kind of amino-based molecule to be contained in the particles.

In the case where the kind of amino-based molecule-containing particles is selected for each patient, for example, using a cancer cell sample collected from the cancer lesions in the patient, the anticancer activity (e.g. activity to damage cancer cells) of a series of amino-based molecule-containing particles is examined to determine which particles exhibit a high anticancer activity. If amino-based molecule-containing particles which exhibit an anticancer activity significantly higher than a known anticancer agent and/or control polymer particles containing no amino-based molecules are found, the particles may be selected as particles to be administered to the above-mentioned patient. If a plurality of kinds of amino-based molecule-containing particles are determined to be effective in the *in vitro* test, all the determined amino-based molecules may be incorporated into the same or different particles, and the prepared particles may be administered to the patient. Tailor-made medicine in which treatment is optimized for individual patients can be achieved by selecting amino-based molecule-containing particles which are expected to have the highest effect in a patient and administering the selected particles to the patient. As a method for evaluation of anticancer activity using a cell sample, various methods such as MTT assay are known, and any of known methods may be employed.

The level of anticancer activity can be modulated by selection of polymerization process of polymer particles. For example, as shown in Fig. 6, in the case of alanine-containing particles, those produced by the novel synthesis method of nanoparticles have a higher effect to inhibit proliferation of 4 types of cancers, T-cell lymphoma, colon cancer,

pancreatic cancer and liver cancer. Glycine-containing particles are extremely effective against T-cell lymphoma, and also very effective against colon cancer, pancreatic cancer and liver cancer. This anticancer activity of glycine-containing particles is further increased by producing the particles by the novel synthesis method of nanoparticles. The anticancer activity of proline-containing particles tends to be increased by producing them by the novel synthesis method of nanoparticles. Selection of polymerization process of particles may also be carried out in a similar manner to selection of the kind of amino-based molecules described above.

The cell-damaging agent of the present invention may be composed only of amino-based molecule-containing particles. In cases where the agent is used as a pharmaceutical, a known carrier(s) such as an excipient(s), diluent(s), etc. may be mixed with the particles to prepare the agent in a formulation suitable for each administration mode. The agent may comprise a single kind of particles, or two or more kinds of particles may be used in combination.

Examples of the method for administration of the particles include parenteral administration such as subcutaneous, intramuscular, intraperitoneal, intraarterial, intravenous and intrarectal administration and oral administration. Specifically, for example, the amino-based molecule-containing particles may be suspended in physiological buffered saline and parenterally administered by injection or the like, or may be orally administered as a capsule or syrup. In cases where the particles are administered to livestocks, poultry or farmed fish, the particles may be added to feed and orally administered. In cases where the particles are used in therapy or prophylaxis of cancers, the particles may be topically administered to tumor and its vicinity. In cases where the particles are used for sterilization of medical instruments etc., the particles may be, for example, dispersed in water, an alcohol solvent or the like and medical instruments etc. may be immersed therein. By administering the amino-based molecule-containing particles to a living body or bringing them into contact with instruments or the like, the particles are brought into contact with bacteria to be inhibited, thereby attaining inhibition of the growth of bacteria.

In cases where amino-based molecule-containing particles are used in therapy and/or prophylaxis of infections, the dose of the particles may be usually, but not limited to, about 0.41 g to 100 g, e.g. about 0.01 g to 25 g, per once per adult. In the case of Gram-negative bacterial infections, the dose of the particles may be, but not limited to, about 0.1 g to 200 g, e.g. about 0.5 g to 50 g, per once per adult. The level (MIC value and MBC value) of antibacterial activity of the amino-based molecule-containing particles also varies depending on the kind of bacteria. The particles can exhibit antibacterial activity against Gram-positive bacteria at a particle concentration of approximately 1 μg/ml to 1000 μg/ml, and against Gram-negative bacteria at a particle concentration of approximately 50 μg/ml to 7 mg/ml.

In cases where amino-based molecule-containing particles are used for anticancer purposes, the dose is appropriately selected depending on the size of tumor, symptoms and the like. The dose of the particles may be usually, but not limited to, about 10 mg to 200 g, particularly about 100 mg to 50 g, per once per adult.

Animals to be treated with amino-based molecule-containing particles for the purpose of therapy and/or prophylaxis of a cancer(s) are not restricted, and are preferably mammals such as humans, dogs, cats, rabbits, hamsters, monkeys, mice, horses, sheep, cows and the like. These animals to be treated are usually in need of therapy and/or prophylaxis of a cancer(s). For example, an individual diagnosed with cancer is an animal in need of therapy of cancer, and amino-based molecule-containing particles may be administered to such an animal for the purpose of cancer therapy by damaging cancer cells. In those having genetic factors and hence being considered to be at a high risk of developing cancer or those in which cancer has been cured, prevention of cancer development or recurrence is strongly demanded, and amino-based molecule-containing particles may be administered to such animals for the purpose of prophylaxis of cancer.

EXAMPLES

The present invention will now be described more concretely by way of examples. However, the present invention is not restricted to the examples below.

1. Production of Amino Acid-Containing Nanoparticles

Using basic amino acids (arginine, histidine, lysine), acidic amino acids (aspartic acid, glutamic acid) and neutral amino acids (alanine, glycine, leucine, valine, isoleucine, serine, threonine, phenylalanine, tryptophan, tyrosine, cystine, methionine, glutamine, asparagine, proline), cyanoacrylate polymer particles containing each amino acid were produced.

(1) Dex-Amino Acid Incorporating System (Production Example 1)

In 10 mL of 0.01N HCl, 20 mg of amino acid and 100 mg of dextran 70K or 60K were dissolved, and the pH of the solution was adjusted to pH=2 as needed using 1N hydrochloric acid. Concerning Cys and Met, the pH was adjusted to pH=3 using 0.001N HCl solution.

(2) Amino Acid Alone Incorporating System (Production Example 2)

In 10 mL of 0.001N HCl, 100 mg of amino acid was dissolved, and the pH of the solution was adjusted to pH=3 as needed using 1N hydrochloric acid.

Under stirring solutions of (1) and (2), 100 μL of nBCA was added thereto, and each mixture was stirred for 3 hour to allow the polymerization reaction to proceed. The reaction solutions were neutralized (pH7.8) by adding 1N NaOH dropwise thereto, and then stirred for additional 30 minutes, followed by centrifugal filtration of the solutions at 3500 rpm/15 min using a Centriprep (YM-10) filter (MILLIPORE). Distilled water was added to the liquids which did not pass through a Centriprep filter, and the resulting mixtures were filtered by centrifugal filtration again to wash the polymerized particles. This centrifugal washing operation was repeated 4 times in total to obtain various amino acid-containing particles. The particles were prepared as 10 mL colloidal solutions, and 5 mL aliquots of the colloidal solutions were freeze-dried to use them measurement of the weight etc., while the remainders were used for measurement of antibacterial activity.

The average particle diameter and the Zeta-potential of the obtained particles were measured with a commercially available Zetasizer (produced by Malvern Inst.UK). The results of the measurement on the particles of Production Example I (Dex-Amino acid incorporating system) are shown in Table 1, and the results of the measurement on the particles of Production Example 2 (Amino acid alone incorporating system) are shown in Table 2.

Table 1

| | Amino Acid Incorporation Ratio (%) | Average Particle Diameter (nm) | Peak1 (nm) | Peak Width (nm) | Zeta-Potential (-mV) |
|---|---|---|---|---|---|
| Neutral Amino Acid | | | | | |
| 0.01HCl-Ala+D70 | 33.5 | 145; PDI:0.138 | 143 (100%) | 50.5 | 10.9 |
| 0.01HCl-Gly+D70 | 51.6 | 133; PDI:0.029 | 131 (100%) | 33.7 | 16.5 |
| 0.01HCl-Val+D70 | 32.3 | 126; PDI:0.150 | 119 (100%) | 66.4 | 13.4 |
| 0.01HCl-Leu+D70 | 32.7 | 145; PDI:0.228 | 132 (100%) | 62.9 | 10.4 |
| 0.01HCl-Ile+D70 | 29.3 | 122; PDI:0.162 | 113 (99.7%) | 38.8 | 11.8 |
| 0.01HCl-Ser+D70 | 31.1 | 133; PDI:0.090 | 131 (100%) | 47.3 | 14.4 |
| 0.01HCl-Thr+D70 | 28.4 | 147; PDI:0.153 | 144 (99.8%) | 57.2 | 7.69 |
| 0.001HCl-Cys+D70 | 26.6 | 417; PDI:0.048 | 466 (100%) | 88.7 | 9.07 |
| 0.001HCl-Met+D70 | 29.1 | 142; PDI:0.048 | 143 (100%) | 45.0 | 8.32 |
| 0.01HCl-Phe+D70 | 22.9 | 136; PDI:0.219 | 122 (100%) | 46.2 | 5.42 |
| 0.01HCl-Tyr+D70 | 27.6 | 122; PDI:0.078 | 117 (100%) | 40.4 | 5.84 |
| 0.01HCl-Trp+D70 | 31.4 | 123; PDI:0.062 | 120 (100%) | 32.2 | 13.5 |
| 0.01HCl-Asn+D70 | 23.9 | 144; PDI:0.162 | 139 (98.6%) | 42.4 | 10.2 |
| 0.01HCl-Gln+D70 | 27.4 | 137; PDI:0.198 | 127 (98.8%) | 56.0 | 8.6 |

(continued)

| | Amino Acid Incorporation Ratio (%) | Average Particle Diameter (nm) | Peak1 (nm) | Peak Width (nm) | Zeta-Potential (-mV) |
|---|---|---|---|---|---|
| Neutral Amino Acid | | | | | |
| 0.01HCl-Pro+D70 | 26.0 | 114; PDI:0.095 | 106 (100%) | 42.1 | 14.7 |
| 0.01HCl-Gly+D60 | 52.0 | 175; PDI:0.052 | 188 | 52.1 | 21.5 |
| 0.01HCl-Ala+D60 | 39.6 | 176; PDI:0.047 | 189 | 52.2 | 19.5 |
| Acidic Amino Acid | | | | | |
| 0.01HCl-Asp+D70 | 28.5 | 138; PDI:0.151 | 137 (100%) | 72.5 | 10.6 |
| 0.01HCl-Glu+D70 | 21.7 | 135; PDI:0.233 | 117(98.6%) | 55.0 | 9.83 |
| 0.01HCl-Asp+D60 | 24.3 | 140; PDI:0.128 | 163 | 65.1 | 12.3 |
| Basic Amino Acid | | | | | |
| 0.01HCl-Lys+D70 | 36.0 | 153; PDI:0.143 | 160 (100%) | 69.9 | 20.2 |
| 0.01HCl-Arg+D70 | 31.6 | 135; PDI:0.117 | 136 (100%) | 54.3 | 24.7 |
| 0.01HCl-His+D70 | 35.3 | 286; PDI:0.148 | 303 (100%) | 89.5 | 10.9 |
| 0.01HCl-Arg+D60 | 32.7 | 307; PDI:0.096 | 341 | 95.2 | 15.8 |
| Control (comprising no amino acid) | | | | | |
| 0.01HCl-D70 | - | 147; PDI:0.216 | 136 (100%) | 58.9 | 26.6 |

Table 2

| | Amino Acid Incorporation Ratio (%) | Average Particle Diameter (nm) | Peak1 (nm) | Peak Width (nm) | Zeta-Potential (-mV) |
|---|---|---|---|---|---|
| 0.01HCl-Ala | 49.9 | 226; PDI:0.022 | 235 (100%) | 35.5 | 38.7 |
| 0.01HCl-Gly | 61.6 | 554; PDI:0.0167 | 547 (100%) | 37.4 | 48.1 |
| 0.01HCl-Pro | 48.8 | 287; PDI:0.315 | 273 (100%) | 66.4 | 4.04 |
| 0.01HCl-His | 51.2 | 320; PDI:0.228 | 332 (100%) | 52.9 | 4.51 |
| 0.01HCl-D70 | - | 147; PDI:0.216 | 136 (100%) | 58.9 | 26.6 |

2. Antibacterial Activity of Amino Acid-Containing Particles

2.1 Antibacterial Activity of Particles of Dex-Amino Acid Incorporating System

(Part 1)

The particles produced by Dex-Amino acid incorporating system of Production Example 1 was examined for their antibacterial activity (minimum inhibitory concentration (MIC)) against various bacterial strains. Ampicillin (ABPC) and vancomycin (VCM) were used as positive controls. The measurement was carried out in accordance with a broth microdilution method (NCCLS). That is, 256 μg/ml of ABPC and VCM solutions and 6.4 mg/ml of particle suspension were prepared as undiluted solutions (1-fold dilution), and each undiluted solution was 2-fold serially diluted up to 2048-fold to prepare 12 concentrations in total, which were used for assessing the antibacterial activity. These serial dilutions were placed in a 96-well plate, and a bacterial strain was added to each well at an amount of $10^5$ CFU/ml, followed by incubation at 35°C for 18 hours. In the case where turbidity was visually observed, it was judged that the bacterial strain grew. The lowest concentration at which the bacterial growth was not observed was defined as MIC. The results are shown in Table 3. In addition, the results shown in Table 3 are also shown as a graph in Fig. 1 (*Staphylococcus aureus*) and Fig. 2 (*Enterococcus*).

Table 3

| | MRSA N315 | MRSA JCM8703 | MSSA ATCC6538 | MSSA JCM2874 | VRE NCTC12201 | VRE GTC02000 | VSE JCM5804 |
|---|---|---|---|---|---|---|---|
| Nanoparticles | MIC (μg/ml) | (μg/ml) | (μg/ml) | (μg/ml) | (μg/ml) | (μg/ml) | (μg/ml) |
| D70 | 100 | 100 | 100 | 100 | 100 | 100 | 100 |
| | | | | | | | |
| Ala+D70 | 75 | 75 | 75 | 75 | 75 | 75 | 75 |
| Gly+D70 | 25 | 25 | 25 | 25 | 25 | 25 | 25 |
| Val+D70 | 75 | 75 | 75 | 75 | 75 | 75 | 75 |
| Leu+D70 | 100 | 100 | 100 | 100 | 100 | 100 | 100 |
| Ile+D70 | 75 | 75 | 75 | 75 | 75 | 75 | 75 |
| Ser+D70 | 50 | 50 | 50 | 50 | 50 | 50 | 50 |
| Thr+D70 | 32.5 | 32.5 | 32.5 | 32.5 | 32.5 | 32.5 | 32.5 |
| Cys+D70 | 100 | 100 | 100 | 100 | 100 | 100 | 100 |
| Met+D70 | 150 | 150 | 150 | 150 | 150 | 150 | 150 |
| Phe+D70 | 75 | 75 | 75 | 75 | 75 | 75 | 75 |
| Tyr+D70 | 75 | 75 | 75 | 75 | 75 | 75 | 75 |
| Trp+D70 | 75 | 75 | 75 | 75 | 75 | 75 | 75 |
| Asp+D70 | 75 | 75 | 75 | 75 | 75 | 75 | 75 |
| Glu+D70 | 50 | 50 | 50 | 50 | 50 | 50 | 50 |
| Asn+D70 | 32.5 | 32.5 | 32.5 | 32.5 | 32.5 | 32.5 | 32.5 |
| Gln+D70 | 75 | 75 | 75 | 75 | 75 | 75 | 75 |
| Gys+D70 | 100 | 100 | 100 | 100 | 100 | 100 | 100 |
| Arg+D70 | 100 | 100 | 100 | 10 | 50 | 50 | 50 |
| His+D70 | 12.5 | 12.5 | 12.5 | 12.5 | 6.25 | 12.5 | 12.5 |
| Pro+D70 | 50 | 50 | 50 | 50 | 100 | 50 | 100 |
| Aminoacid alone; MIC >400μg/ml | | | | | | | |
| ABPC | 64 | 128 | 0.125 | 1 | 2 | 1 | 1 |
| VCM | 1 | 2 | 1 | 1 | 64 | 16 | 1 |

The amino acid-containing particles showed an antibacterial activity at the same level as or higher than the known antibacterial nanoparticles comprising no amino acids. The antibacterial activity of the amino acid-containing particles were equally effective against not only sensitive bacteria but also resistant bacteria such as MRSA and VRE. In particular, glycine-, asparagine- and histidine-containing particles were confirmed to have a higher antibacterial activity than other amino acid-containing particles.

2.2 Antibacterial Activity of Particles of Dex-Ammo Acid Incorporating System

(Part 2)

The particles produced by Dex-Ammo acid incorporating system of Production Example 1 were examined for their antibacterial activity (minimum inhibitory concentration (MIC)) against various bacterial strains. Ampicillin (ABPC) and levofloxacin (LVFX) were used as positive controls. The measurement was carried out m the same manner as in 2.1 above, in accordance with a broth microdilution method (NCCLS). The results are shown in Table 4.

Table 4-1

| Antibacterial Activity of Nanoparticles (MIC, values are in μg/ml) | | | | | | | |
|---|---|---|---|---|---|---|---|
| | MSSA (ATCC6538) | MSSA (JCM2874) | MRSA (N315) | MRSA (JCM8703) | VSE (JCM5804) | VRE (NCTC12201) | VRE (GTC02000) |
| | *S. aureus* | *S. aureus* | *S. aureus* | *S. aureus* | *Enterococcus* | *Enterococcus* | *Enterococcus* |
| | Gram-positive coccus | Gram-positive coccus | Gram-positive coccus | Gram-positive coccus | Gram-positive coccus | Gram-positive coccus | Gram-positive coccus |
| Gly+D60 | 63 | 63 | 125 | 63 | 63 | 31 | 31 |
| Arg+D60 | 63 | 63 | 125 | 63 | 63 | 63 | 63 |
| Asp+D60 | 63 | 63 | 63 | 63 | 31 | 31 | 31 |
| Ala+D60 | 63 | 63 | 63 | 63 | 63 | 31 | 31 |
| ABPC | 0.25> | 2 | >128 | >128 | | | |
| LVFX | | | | | 16 | 0.5 | 2 |

Table 4-2

| Antibacterial Activity of Nanoparticles (MIC, values are in μg/ml) | | | | | | | |
|---|---|---|---|---|---|---|---|
| | E. coli (JCM5491) | E.coli (ATCC8739) | E.coliO157 H7 (RIMD0509939) | P. aeruginosa (JCM6119) | S. bongori (ATCC43975) | S. arizonae (ATCC13314) | K. pneumoniae (JCM1662) |
| | *E. coli* | *E. coli* | *E. coli* | *P. aeruginosa* | *Salmonella* | *Salmonella* | *Klebsiella* |
| | Gram-negative bacillus | Gram-negative bacillus | Gram-negative bacillus | Gram-negative bacillus | Gram-negative bacillus | Gram-negative bacillus | Gram-negative bacillus |
| Gly+D60 | 2000 | 4000 | 4000 | 4000 | 2000 | 1000 | 1000 |
| Arg+D60 | 1000 | 1000 | 1000 | 1000 | 1000 | 1000 | 500 |
| Asp+D60 | 2000 | 4000 | 4000 | 4000 | 1000 | 1000 | 500 |
| Ala+D60 | 2000 | 4000 | 4000 | 4000 | 1000 | 1000 | 500 |
| ABPC | | | | | | | |
| LVFX | 0.063> | 0.063> | 0.063> | 0.5 | 0.063> | 0.063> | 0.063> |

Table 4-3

| Antibacterial Activity of Nanoparticles (MIC, values are in μg/ml) | | | | | | | |
|---|---|---|---|---|---|---|---|
| | K. pneumoniae (BAA-1141) | C. diphtheriae (ATCC13812) | S. anginosus (NCTC10713) | S. pneumoniae (ATCC6301) | S. pyogenes (ATCC12344) | P. anaerobius (ATCC27337) | P. acnes (ATCC11827) |
| | *Klebsiella* | *C. diphtheriae* | oral streptorocci | *S. pneumoniae* | *S. pyoygenes* | Peptostreptococcus | *Propionibacterium acnes* |
| | Gram-negative bacillus | Gram-positive bacillus | Gram-positive coccus | Gram-positive coccus | Gram-positive coccus | Gram-positive coccus | Gram-positive bacillus |
| Gly+D60 | 2000 | 31 | 31 | 16 | 8 | 8 | 8 |
| Arg+D60 | 1000 | 31 | 63 | 16 | 16 | 8 | 8 |
| Asp+D60 | 1000 | 16 | 31 | 16 | 16 | 8 | 8 |
| Ala+D60 | 1000 | 16 | 31 | 16 | 16 | 8 | 8 |
| ABPC | | 0.25> | 0.25> | 0.25> | 0.25> | 0.25> | 0.25> |
| LVFX | 0.5 | | | | | | |

Table 4-4

| Antibacterial Activity of Nanoparticles(MIC, values are in μg/ml) | | | | | | | |
|---|---|---|---|---|---|---|---|
| | M. tuberculosis (ATCC27294) | M tuberculosis (H-17) | M. tuberculosis (ATCC35812) | M. tuberculosis (ATCC35838) | M. tuberculosis (ATCC35837) | M. tuberculosis (ATCC35827) | M. tuberculosis (ATCC35820) |
| | tubercle bacillus sensitive | tubercle bacillus multiresistant | tubercle bacillus INH resistant | tubercle bacillus RFP resistant | tubercle bacillus EB resistant | tubercle bacillus KM resistant | tubercle bacillus SM resistant |
| | Gram-positive bacillus | Gram-positive bacillus | Gram-positive bacillus | Gram-positive bacillus | Gram-positive bacillus | Gram-positive bacillus | Gram-positive bacillus |
| Gly+D60 | 62.5 | 62.5 | 62.5 | 62.5 | 12.5 | 62.5 | 62.5 |
| Arg+D60 | 12.5 | 125 | 12.5 | 125 | 125 | 125 | 125 |
| Asp+D60 | 62.5 | 62.5 | 62.5 | 62.5 | 125 | 62.5 | 62.5 |
| Ala+D60 | 62.5 | 62.5 | 62.5 | 62.5 | 125 | 62.5 | 62.5 |

Table 4-5

| Antibacterial Activity of Nanoparticles (MIC, values are in μg/ml) | | | |
|---|---|---|---|
| | M. avium (ATCC25291) | M. interacellulare (ATCC13950) | M. kansasii (ATCC12478) |
| | non-tuberculous mycobacteriu m | non-tuberculous mycobacteriu m | non-tuberculous mycobacteriu m |
| | Gram-positive bacillus | Gram-positive bacillus | Gram positive bacillus |
| Gly+D60 | 250 | 62.5 | 12.5 |
| Arg+D60 | 500 | 12.5 | 12.5 |
| Asp+D60 | 250 | 62.5 | 125 |
| Ala+D60 | 250 | 62.5 | 125 |

It was confirmed that the amino acid-containing particles could inhibit the growth of various bacteria including not only Gram-positive bacteria but also Gram-negative bacteria

3 Antibacterial Activity of Amino Acid-Containing Particles (Companson between Dex-Amino Acid Incorporating System and Ammo Acid Alone Incorporating System)

Four types (Gly, Ala, His, Pro) of nanoparticles of Production Example 2 prepared without using dextran were assessed for their antibacterial activity in the same manner as described above, and their activity was compared to the antibacterial activity of nanoparticles of Production Example 1. The results are shown in Table 5. In addition, the results shown in Table 5 are also shown as a graph in Fig. 3 *(Staphylococcus aureus)* and Fig. 4 *(Enterococcus).*

Table 5

| | MRSA N315 | MRSA JCM8703 | MSSA ATCC5S38 | MSSA JCM2874 | VRE NCTC12201 | VRE GTC02000 | VSE JCM5804 |
|---|---|---|---|---|---|---|---|
| Nanoparticles | (μg/ml) | (μg/ml) | (μg/ml) | (μg/ml) | (μg/ml) | (μg/ml) | (μg/ml) |
| D70 | 100 | 100 | 100 | 100 | 100 | 100 | 100 |
| Gly | 5 | 5 | 5 | 5 | 2.5 | 5 | 2.5 |
| Gly+Dex70 | 25 | 25 | 25 | 25 | 25 | 25 | 25 |
| Ala | 50 | 50 | 50 | 50 | 50 | 50 | 50 |
| Ala+Dex70 | 75 | 75 | 75 | 75 | 75 | 75 | 75 |
| His | 10 | 10 | 10 | 10 | 5 | 5 | 5 |
| His+Dex70 | 12.5 | 12.5 | 12.5 | 12.5 | 6.255 | 12.5 | 12.5 |
| Pro | 50 | 50 | 50 | 50 | 100 | 50 | 100 |
| Pro+Dex70 | 50 | 50 | 50 | 50 | 100 | 50 | 00 |
| ABPC | 64 | 128 | 0.125 | 1 | 2 | 1 | 1 |
| VCM | 1 | 2 | 1 | 1 | 64 | 16 | 1 |

All the four types of particles containing amino acid alone showed an antibacterial activity at the same level as or higher than the nanoparticles of Production Example 1 prepared using dextran. The antibacterial activity of nanoparticles could be improved by not using dextran, which was used as a polymerization initiator/stabilizer.

4. Observation of Bacteriolysis Caused by Amino Acid-Containing Particles

Gram-positive bacteria were treated with the amino acid-containing nanoparticles (0.01 HCl-Gly+Dex70) synthesized in Production Example 1, and the morphological change of the treated bacteria was observed with an electron microscope. Treatment with the nanoparticles was carried out as follows.

The above-described particles were suspended in physiological saline to prepare a particle suspension (6 μg/ml). Using a commercially available 24-well cell culture plate, 1 ml of the particle suspension was added to $10^5$ to $10^6$ cells/1 ml per well of VRE, and the particles and bacterial cells were suspended together in the wells at room temperature for I hour. Thereafter, 1 ml/well of physiological saline was added thereto, and the plate was shaken twice or 3 times, followed by removing a washing fluid by aspiration. This washing operation was repeated another twice to carry out the washing 3 times in total. The washed bacterial cells were observed with a scanning election microscope (SEM).

SEM images of VRE NCTC12201 taken 1 hour, 3 hours and 6 hours after the treatment are shown in Fig. 5. It was observed that, upon treatment with amino acid-containing nanoparticles, the particles adhered to the bacterial cell surface, and that swelling and lysis of the bacterial cells occurred.

5. *In Vivo* Antibacterial Activity of Amino Acid-Containing Nanoparticles

The amino acid-containing nanoparticles were examined for their antibacterial activity in VRE-infected mice ($10^8$ cfu/0.5ml per mouse of VRE was orally inoculated). Glycine-containing nanoparticles were orally administered to mice at a concentration of 1 mg/ml three days before or one hour after VRE inoculation. The amount of VRE in a mouse body was confirmed by measuring the number of VRE bacterial cells per 1 g of feces. As a result, the number of bacterial cells remarkably decreased both in the mice which received the administration before infection and in the mice which received the administration after infection, when compared to the control mice groups to which physiological saline was administered. Thus, it was confirmed that the amino acid-containing particles have *in vivo* prophylactic and therapeutic effects on infections.

6. Anticancer Activity of Amino Acid-Containing Particles (Comparison between Dex-Amino Acid Incorporating System and Amino Acid Alone Incorporating System)

The amino acid-containing particles of Production Example 1 and Production Example 2 were assessed for their cell-damaging activity by cell proliferation assay based on MTT method using various cell lines treated with the amino acid-containing particles. The assay was performed using MTT assay kit (Roche). The cell lines used were H9 (human malignant lymphoma), HCT116 (human colon cancer), MIAPaCa (human pancreatic cancer) and HepG2 (human liver cancer).

The cell lines were each adjusted to a cell density of 1 x $10^5$ cell/mL in RPMI1640 medium supplemented with 5% fetal bovine serum. One-hundred-microliter aliquots of cell suspensions were placed into wells of 96-well cell culture plate (CELLSTAR (registered trademark)), and cultured in a $CO_2$ incubator at 37°C for 24 hours. The nanoparticle suspension whose concentration was adjusted with distilled water was added dropwise to the wells (the concentration of nanoparticles during the treatment was 5 μg/ml for H9, and 10 μg/ml for the other cell lines), and the particle treatment was performed for 24 hours in a $CO_2$ incubator. To each well, 10 μL of MTT labeling reagent was added (final concentration of MTT: 0.5 mg/mL/well), and the cells were cultured in a $CO_2$ incubator for 4 hours. One hundred microliters of Solubilization solution was added to each well, and cells were cultured in a $CO_2$ incubator overnight, followed by measuring absorbance at 550 nm. The reference wavelength was set at 700 nm.

The results are shown in Fig. 6. The level of cell-damaging activity varied depending on the kind of amino acid and the kind of cancer. Particles containing Gly, Asn, Gln, or His showed a strong anticellular effect against H9 malignant lymphoma. Strong anticellular effects were also shown by particles containing Gly, Asn, or His against HCT116 colon cancer; by particles containing Gly or His against MIAPaCa pancreatic cancer; and by particles containing His against HepG2 liver cancer. In some cases, particles of Production Example 2 prepared by amino acid alone incorporating system showed a higher cell-damaging activity than particles of Production Example 1 prepared by a conventional method. For example, in the cases of glycine and alanine, particles of amino acid alone incorporating system showed a higher cell-damaging activity against all the four cell lines. In the case of threonine, particles of amino acid alone synthetic system showed a higher cell-damaging activity against a part of the cell lines (H9, HCT116). No cell-damaging activity was observed when cell lines were treated with amino acid alone which was not contained in particles at an amino acid concentration of 200 μg/ml or more.

7. Confirmation of Adhesion of Amino Acid-Containing Particles to Tumor Cells

H9 cells were treated with the amino acid-containing nanoparticles (0.01HCl-Gly+Dex70) synthesized in Production Example 1, and the treated cells were observed with an electron microscope. Treatment with nanoparticles was carried out in the same manner as in 6 above. Fig. 7 shows SEM images of a H9 cell two hours after addition of

nanoparticles. The cell maintained its spherical shape. Free nanoparticles were observed around the cell, and nanoparticles which adhered to the cell surface were observed (Fig. 7A). When the cell surface was observed at a higher magnification, folds were degenerated and smoothening occurred (Fig. 7B), and a plurality of nanoparticles which adhered to the smoothened site were observed (Fig. 7C).

8. Investigation of *In Vivo* Toxicity

(1) Amino acid-containing nanoparticles were administered (oral administration, intravenous injection, intraperitoneal administration, subcutaneous administration) to healthy mice and nude mice at a concentration of 1 mg/ml. The mice were observed up to 6 months after administration to find that they normally survived without any abnormalities. It was confirmed that amino acid-containing nanoparticles did not injure normal cells.

(2) Glycine-containing nanoparticles were administered to 6-week-old ICR female mice (20 g to 24 g body weight) in a single dose of 50 mg/body orally, or in a single dose of 10 mg/body intraperitoneally, intravenously via tail vein, subcutaneously, or cutaneous application (each administration group was composed of 5 mice), and the mice were checked every day for body weight change, feces and behaviors during 8 days after administration. The mice were compared to the mice group to which physiological saline was administered to find no abnormalities in the treated mice.

## Claims

**1.** A method for production of cyanoacrylate polymer particles, said method comprising anionically polymerizing a cyanoacrylate monomer(s) in the presence of at least one selected from the group consisting of amino acids, amino acid derivatives, and oligomers and polymers thereof, and substantially in the absence of a saccharide and substantially in the absence of a polysorbate.

**2.** The method according to claim 1, wherein the anionic polymerization is carried out in the presence of at least one selected from the group consisting of amino acids, amino acid derivatives, and oligomers thereof.

**3.** The method according to claim 2, wherein the anionic polymerization is carried out in the presence of an amino acid(s).

**4.** The method according to any one of claims 1 to 3, wherein said amino acid is at least one selected from the group consisting of arginine, histidine, lysine, aspartic acid, glutamic acid, alanine, glycine, leucine, valine, isoleucine, serine, threonine, phenylalanine, tryptophan, tyrosine, cystine or cysteine, glutamine, asparagine, proline, methionine, β-alanine, γ-aminobutyric acid, carnitine, γ-aminolevulinic acid, and γ-aminovaleric acid.

**5.** The method according to claim 1 or 2, wherein said amino acid derivative is at least one selected from the group consisting of creatine, ornithine, thyroxine, desmosine, hydroxyproline, hydroxylysine, phosphoserine, theanine, kainic acid, tricholomic acid, and sarcosine.

**6.** A cell-damaging agent comprising as an effective ingredient cyanoacrylate polymer particles whose average particle diameter is less than 1000 nm, said particles containing at least one selected from the group consisting of amino acids, amino acid derivatives, and oligomers and polymers thereof.

**7.** The cell-damaging agent according to claim 6, which comprises as an effective ingredient the particles containing at least one selected from the group consisting of amino acids, amino acid derivatives, and oligomers thereof.

**8.** The cell-damaging agent according to claim 7, which comprises as an effective ingredient the particles containing an amino acid(s).

**9.** The cell-damaging agent according to any one of claims 6 to 8, wherein said amino acid is at least one selected from the group consisting of arginine, histidine, lysine, aspartic acid, glutamic acid, alanine, glycine, leucine, valine, isoleucine, serine, threonine, phenylalanine, tryptophan, tyrosine, cystine or cysteine, glutamine, asparagine, proline, methionine, β-alanine, γ-aminobutyric acid, carnitine, γ-aminolevulinic acid, and γ-aminovaleric acid.

**10.** The cell-damaging agent according to claim 6 or 7, wherein said amino acid derivative is at least one selected from

the group consisting of creatine, ornithine, thyroxine, desmosine, hydroxyproline, hydroxylysine, phosphoserine, theanine, kainic acid, tricholomic acid, and sarcosine.

**11.** The cell-damaging agent according to claim 8, which comprises as an effective ingredient cyanoacrylate polymer particles containing at least one selected from the group consisting of glycine, asparagine and histidine.

**12.** The cell-damaging agent according to any one of claims 6 to 11, wherein said cell is bacterial cell.

**13.** The cell-damaging agent according to claim 12, wherein said cell is Gram-positive bacterial cell.

**14.** The cell-damaging agent according to any one of claims 6 to 13, wherein said particles are those produced by anionically polymerizing a cyanoacrylate monomer(s) in the presence of at least one selected from the group consisting of amino acids, amino acid derivatives, and oligomers and polymers thereof, and substantially in the absence of a saccharide and substantially in the absence of a polysorbate.

**15.** The cell-damaging agent according to any one of claims 6 to 13, wherein said particles are those produced by anionically polymerizing a cyanoacrylate monomer(s) in the presence of at least one selected from the group consisting of amino acids, amino acid derivatives, and oligomers and polymers thereof, and in the presence of at least one selected from the group consisting of saccharides and polysorbates.

**16.** The cell-damaging agent according to claim 15, wherein said saccharide is at least one selected from the group consisting of monosaccharides having a hydroxy group(s), disaccharides having a hydroxy group(s) and polysaccharides having a hydroxy group(s).

**17.** The cell-damaging agent according to any one of claims 6 to 16, wherein the average particle diameter of said particles is 20 nm to 700 nm.

**18.** A method for modulating the cell-damaging activity of the cell-damaging agent according to claim 6, **characterized in that** the cell-damaging activity of the cyanoacrylate polymer particles is modulated by at least one of: selection of the kind of amino acid(s), amino acid derivative(s), and/or oligomer(s) and/or polymer(s) thereof to be contained in the particles; and selection of the polymerization process of cyanoacrylate polymer particles.

**19.** The method according to claim 18, wherein the polymerization process is selected from the followings:

(1) a process wherein a cyanoacrylate monomer(s) is(are) anionically polymerized in the presence of at least one selected from the group consisting of amino acids, amino acid derivatives, and oligomers and polymers thereof, and substantially in the absence of a saccharide and substantially in the absence of a polysorbate; and
(2) a process wherein a cyanoacrylate monomer(s) is(are) anionically polymerized in the presence of at least one selected from the group consisting of amino acids, amino acid derivatives, and oligomers and polymers thereof, and in the presence of at least one selected from the group consisting of saccharides and polysorbates.

**20.** An antibacterial agent comprising as an effective ingredient cyanoacrylate polymer particles having an average particle diameter of less than 1000 nm, wherein said particles contain at least one selected from the group consisting of amino acids, amino acid derivatives, and oligomers and polymers thereof, and substantially do not contain an active antibacterial ingredient.

**21.** The antibacterial agent according to claim 20, which is an antibacterial agent for a Gram positive bacterium/bacteria, said agent comprising as an effective ingredient said particles which substantially do not contain an active antibacterial ingredient effective against a Gram-positive bacterium/bacteria.

**22.** The antibacterial agent according to claim 20 or 21, which comprises as an effective ingredient said particles containing at least one selected from the group consisting of amino acids, amino acid derivatives, and oligomers thereof.

**23.** The antibacterial agent according to claim 22, which comprises as an effective ingredient said particles containing an amino acid(s).

**24.** The antibacterial agent according to claim 23, which comprises as an effective ingredient said particles containing at least one selected from the group consisting of alanine, glycine, valine, isoleucine, serine, threonine, phenylalanine,

tyrosine, tryptophan, aspartic acid, glutamic acid, asparagine, glutamine, histidine and proline.

**25.** The antibacterial agent according to any one of claims 20 to 24, wherein said particles are those produced by the method according to any one of claims 1 to 5.

**26.** An agent for therapy and/or prophylaxis of a cancer(s), comprising as an effective ingredient particles produced by the method according to any one of claims 1 to 5.

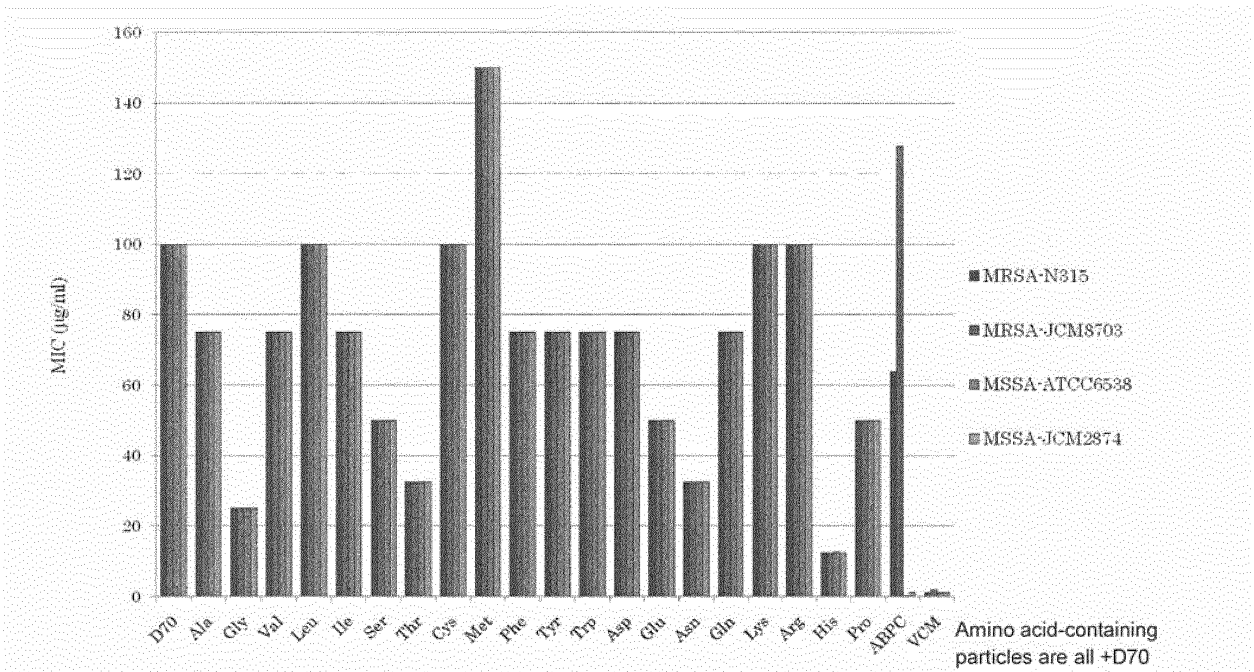
160
140
120
100
80
60
40
20
0
MIC (μg/ml)
MRSA-N315
MRSA-JCM8703
MSSA-ATCC6538
MSSA-JCM2874
D70 Ala Gly Val Leu Ile Ser Thr Cys Met Phe Tyr Trp Asp Glu Asn Gln Lys Arg His Pro ABPC VCM
Amino acid-containing particles are all +D70

**Fig.1**

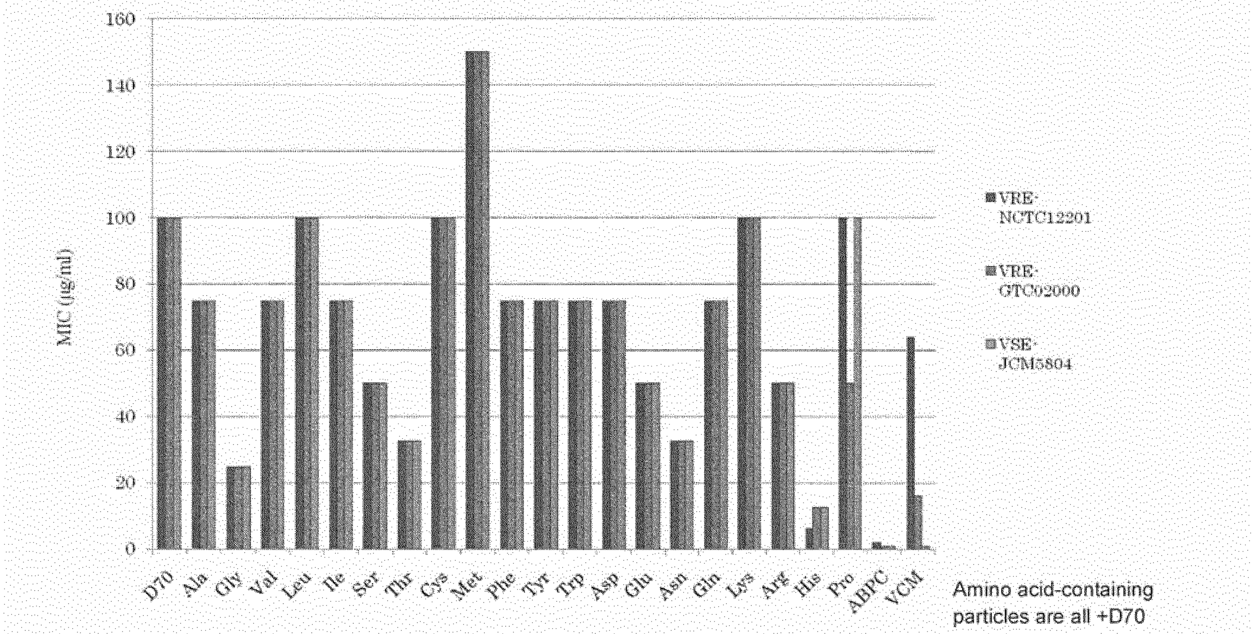
160
140
120
100
80
60
40
20
0
MIC (μg/ml)
VRE-NCTC12201
VRE-GTC02000
VSE-JCM5804
D70 Ala Gly Val Leu Ile Ser Thr Cys Met Phe Tyr Trp Asp Glu Asn Gln Lys Arg His Pro ABPC VCM
Amino acid-containing particles are all +D70

**Fig.2**

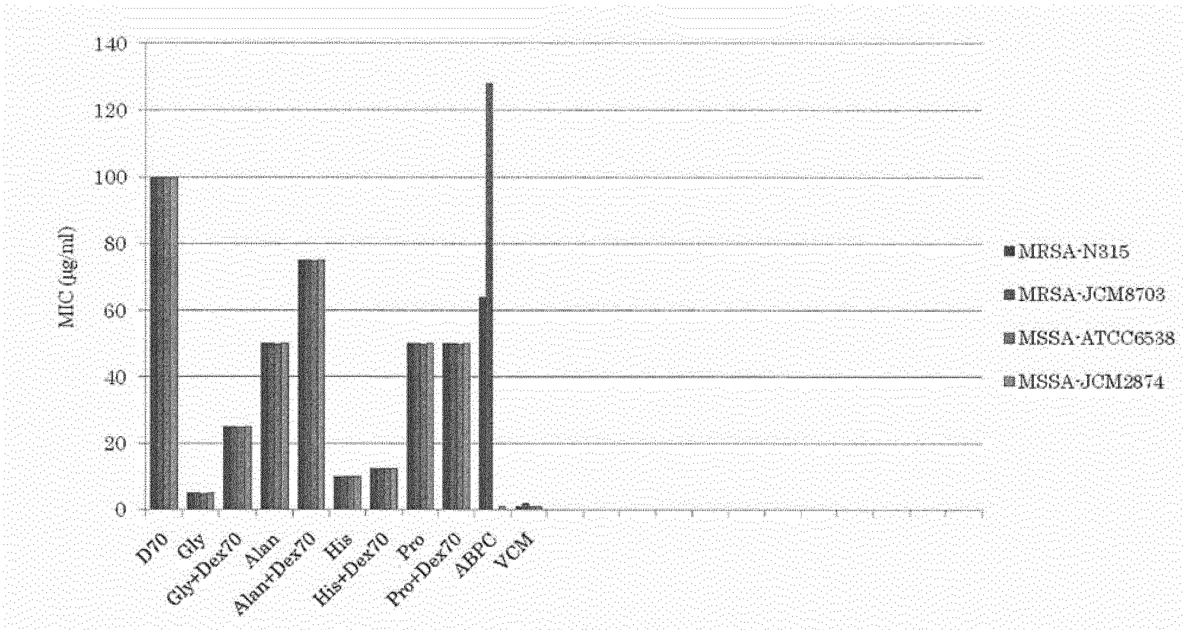
MIC (µg/ml)
140
120
100
80
60
40
20
0
D70
Gly
Gly+Dex70
Alan
Alan+Dex70
His
His+Dex70
Pro
Pro+Dex70
ABPC
VCM
MRSA-N315
MRSA-JCM8703
MSSA-ATCC6538
MSSA-JCM2874

Fig.3

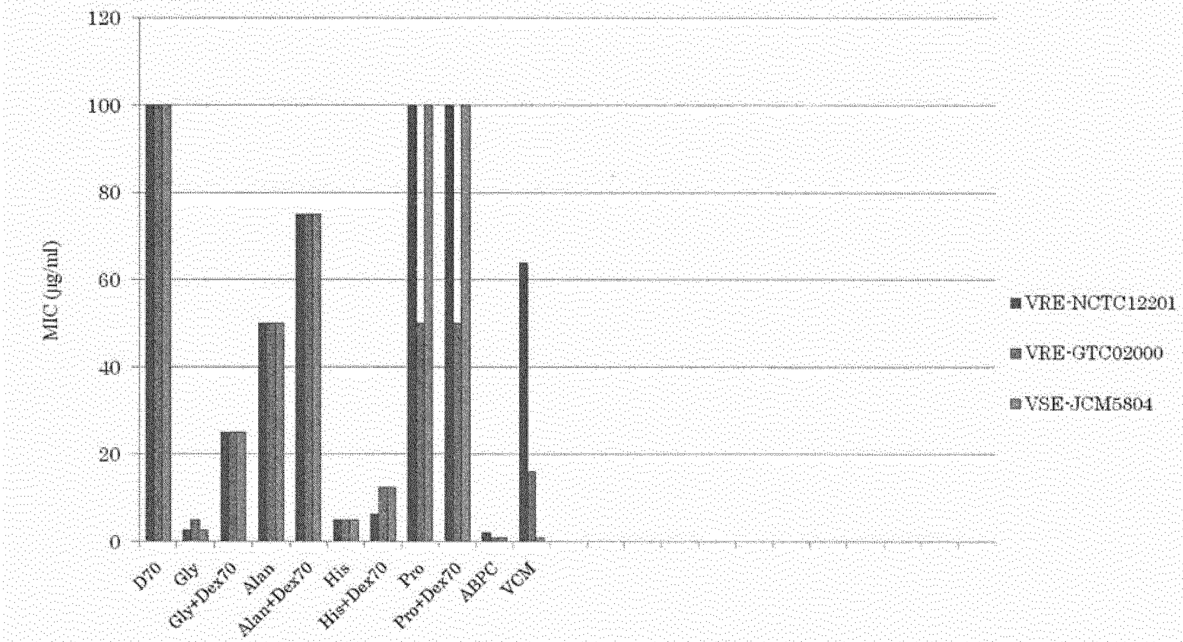
MIC (µg/ml)
120
100
80
60
40
20
0
D70
Gly
Gly+Dex70
Alan
Alan+Dex70
His
His+Dex70
Pro
Pro+Dex70
ABPC
VCM
VRE-NCTC12201
VRE-GTC02000
VSE-JCM5804

Fig.4

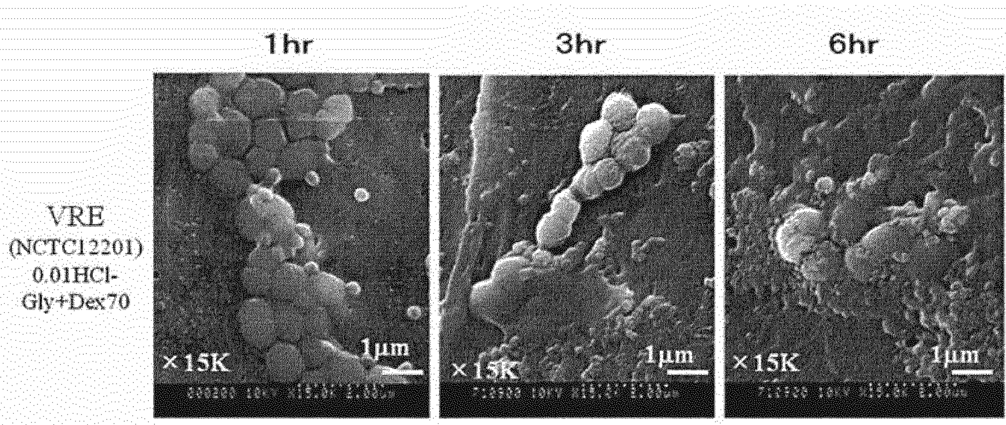


Fig.5

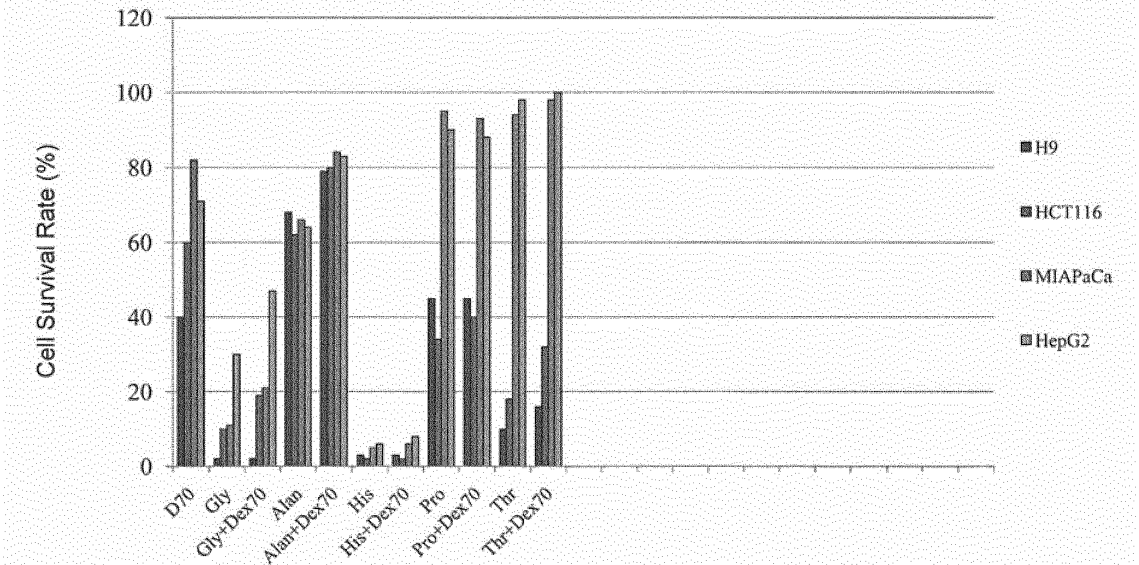


Fig.6

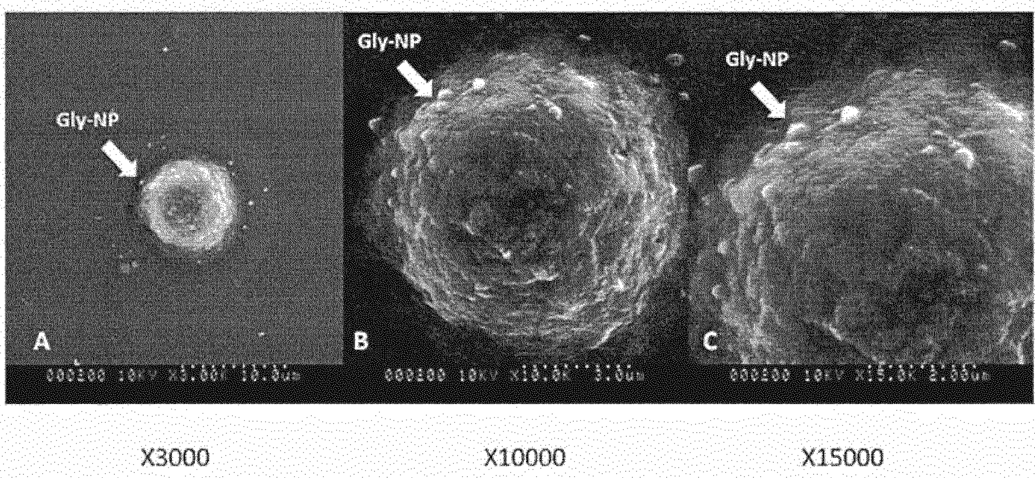


Fig.7

**INTERNATIONAL SEARCH REPORT**

International application No.
PCT/JP2012/058354

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C08F2/44, A61K9/14, A61K31/198, A61K31/401, A61K31/405, A61K31/4172, A61K47/32, A61P31/04, A61P35/00, A61P35/02, C08F120/42, C08F251/00, C08F283/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus(JDreamII)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2009-541462 A (Nanodel Technologies GmbH), 26 November 2009 (26.11.2009), claims; paragraphs [0051], [0052], [0132], [0137]<br>& US 2010/0015165 A1 & EP 1876188 A1<br>& WO 2008/003706 A1 & CA 2654668 A<br>& CN 101484482 A | 1-5,26<br>26 |
| Y | WO 2010/101178 A1 (Yokohama City University), 10 September 2010 (10.09.2010), paragraph [0001]<br>(Family: none) | 26 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>06 June, 2012 (06.06.12) | Date of mailing of the international search report<br>19 June, 2012 (19.06.12) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2012/058354

Continuation of A. CLASSIFICATION OF SUBJECT MATTER
(International Patent Classification (IPC))

*C08F2/44*(2006.01)i, *A61K9/14*(2006.01)i, *A61K31/198*(2006.01)i, *A61K31/401*(2006.01)i, *A61K31/405*(2006.01)i, *A61K31/4172*(2006.01)i, *A61K47/32*(2006.01)i, *A61P31/04*(2006.01)i, *A61P35/00*(2006.01)i, *A61P35/02*(2006.01)i, *C08F120/42*(2006.01)i, *C08F251/00*(2006.01)i, *C08F283/04*(2006.01)i

(According to International Patent Classification (IPC) or to both national classification and IPC)

Form PCT/ISA/210 (extra sheet) (July 2009)

**INTERNATIONAL SEARCH REPORT**

International application No.
PCT/JP2012/058354

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The inventions of claims 1-5 and 26 relate to "a process for producing cyanoacrylate polymer particles" and "a therapeutic and/or prophylactic agent for cancer" which comprises particles produced by the process as an active ingredient; the inventions described in claims 6-19 relate to "a cytotoxic agent" and "a method for modulating a cytotoxic activity"; and the inventions described in claims 20-25 relate to "an antibacterial agent". There is no common or corresponding matter between claim 1 and claim 6 or claim 1 and claim 20 which can be regarded as a special technical feature in the meaning within PCT Rule 13.2, second sentence.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
1-5, 26

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H11503148 A
- JP 2002504526 A
- JP 2008127538 A
- WO 2008126846 A
- JP 2008208070 A
- WO 2009084494 A
- WO 2010101178 A

### Non-patent literature cited in the description

- **CHRISTINE VAUTHIER et al.** *Adv. Drug Deliv. Rev.,* 2003, vol. 55, 519-548